# FASCICULE DE BREVET EUROPEEN

(11) **EP 1 250 285 B1**
(45) Date de publication et mention de la délivrance du brevet: **21.04.2004**
(21) Numéro de dépôt: 01907694.2
(22) Date de dépôt: 25.01.2001
(51) Int. Cl.: C01B 13/36, C01F 17/00, C01G 25/00

(54) **DISPERSION COLLOIDALE AQUEUSE D'UN COMPOSE DE CERIUM ET D'AU MOINS UN AUTRE ELEMENT CHOISI PARMI LES TERRES RARES, DES METAUX DE TRANSITION, L'ALUMINIUM, LE GALLIUM ET LE ZIRCONIUM, PROCEDE DE PREPARATION ET UTILISATION**
COLLOIDALE WÄSSRIGE DISPERSION EINER CERIUMVERBINDUNG UND ZUMINDEST EIN ANDERES ELEMENT AUS DER GRUPPE VON SELTENEN ERDEN, ÜBERGANGSMETALLE, ALUMINIUM, GALLIUM UND ZIRCONIUM, VERFAHREN ZUR HERSTELLUNG UND VERWENDUNG
AQUEOUS COLLOIDAL DISPERSION OF A CERIUM COMPOUND AND AT LEAST ANOTHER ELEMENT SELECTED AMONG RARE EARTHS, TRANSITION METALS, ALUMINIUM, GALLIUM AND ZIRCONIUM, PREPARATION METHOD AND USE

(30) Priorité: 26.01.2000 FR 0001001
(43) Date de publication de la demande: 23.10.2002
(73) Titulaire: Rhodia Electronics and Catalysis, 17041 La Rochelle Cedex (FR)
(72) Inventeur: CHANE-CHING, Jean-Yves, F-95600 Eaubonne (FR)
(74) Mandataire: Dubruc, Philippe
(86) Numéro de dépôt international: PCT/FR2001/000235
(87) Numéro de publication internationale: WO 2001/055029

(56) Documents cités:
- GB-A- 1 603 794
- US-A- 4 231 893
- US-A- 4 606 847
- PATENT ABSTRACTS OF JAPAN vol. 014, no. 090 (C-0691), 20 février 1990 (1990-02-20) & JP 01 301517 A (CATALYSTS & CHEM IND CO LTD), 5 décembre 1989 (1989-12-05)

## Description

La présente invention concerne une dispersion colloïdale aqueuse d'un composé de cérium et d'au moins un autre élément choisi parmi les terres rares autres que le cérium; le titane, le vanadium, le chrome, le manganèse, le fer, le cobalt, le nickel, le cuivre, le zinc, l'aluminium, le gallium et le zirconium.

Les sols de cérium, tout particulièrement les sols de cérium tétravalent, sont bien connus. Par ailleurs, les sols de cérium en combinaison avec un autre élément peuvent présenter un grand intérêt par exemple pour des applications en cosmétique ou dans le domaine des luminophores, et notamment ceux susceptibles de contenir du cérium trivalent. Cependant, dans ces applications, on a besoin de sols qui sont concentrés et qui sont purs.

On connaît par US 4606847 des sols de cérium et d'un autre élément comme l'yttrium ou une terre rare. Toutefois, ces sols présentent encore un taux d'impuretés élevé.

L'objet de l'invention est de résoudre de telles difficultés et donc d'obtenir des sols concentrés et purs, susceptibles notamment de contenir du cérium trivalent.

L'invention concerne donc une dispersion colloïdale aqueuse d'un composé de cérium et d'au moins un autre élément choisi parmi le titane, le vanadium, le chrome, le manganèse, le fer, le cobalt, le nickel, le cuivre, le zinc, l'aluminium, le gallium, le zirconium et le groupe des terres rares constitué par l'yttrium et les éléments de la classification périodique de numéro atomique compris inclusivement entre 57 et 71 à l'exception du cérium, le composé du cérium et de l'élément M étant un oxyde ou un oxyde hydraté, caractérisée en ce qu'elle présente une conductivité d'au plus 5mS/cm.

L'invention concerne aussi un procédé de préparation d'une telle dispersion colloïdale qui est caractérisé en ce qu'on fait réagir avec une base un mélange d'au moins un sel de cérium avec au moins un sel d'un élément M précité et en présence d'un acide en quantité telle que le rapport atomique H⁺/(Ce+M) soit supérieur à 0,1, le pH du milieu réactionnel étant compris entre 7,5 et 9,5; puis on lave et on redisperse dans l'eau le précipité issu de la réaction précédente.

D'autres caractéristiques, détails et avantages de l'invention apparaîtront encore plus complètement à la lecture de la description qui va suivre, ainsi que des divers exemples concrets mais non limitatifs destinés à l'illustrer.

Pour la suite de la description, l'expression dispersion colloïdale ou sol d'un composé de cérium et d'un autre élément précité désigne tout système constitué de fines particules solides de dimensions colloïdales à base d'oxyde et/ou d'oxyde hydraté (hydroxyde) de cérium et de l'autre élément, en suspension dans une phase liquide aqueuse, lesdites espèces pouvant en outre, éventuellement, contenir des quantités résiduelles d'ions liés ou adsorbés tels que par exemple des acétates, des citrates, des nitrates, des chlorures ou des ammoniums. Le pourcentage d'ions liés X ou éventuellement X+Y, exprimé en rapport molaire X/Ce ou (X+Y)/Ce peut varier par exemple entre 0,01et 1,5, plus particulièrement entre 0,01 et 1. On notera que dans de telles dispersions, le cérium et l'autre élément peuvent se trouver soit totalement sous la forme de colloïdes, soit simultanément sous la forme d'ions ou de poly-ions et sous la forme de colloïdes.

Par terre rare on entend les éléments du groupe constitué par l'yttrium et les éléments de la classification périodique de numéro atomique compris inclusivement entre 57 et 71.

Une première caractéristique de la dispersion de l'invention est sa pureté. Cette pureté est mesurée par la conductivité de la dispersion. Cette conductivité est d'au plus 5mS/cm. Elle peut être inférieure à cette valeur et ainsi être d'au plus 2mS/cm et de préférence d'au plus 1mS/cm. Plus particulièrement, elle peut être inférieure à 0,3mS/cm.

Selon une autre caractéristique, la dispersion de l'invention présente une concentration d'au moins 50g/l. Cette concentration est exprimée en oxyde et prenant en compte la somme des oxydes de cérium et de l'autre ou des autres éléments précités. Cette concentration peut être plus particulièrement d'au moins 80g/l.

Une autre caractéristique de la dispersion est qu'elle peut contenir du cérium à l'état d'oxydation III. Dans ce cas, le taux de cérium III est généralement d'au plus 50%. Il est exprimé ici et pour l'ensemble de la description par le rapport atomique CeIII/Ce total. Le taux de cérium III peut être plus particulièrement d'au plus 35%. Par ailleurs, il est de préférence d'au moins 0,5%.

Les dispersions de l'invention sont particulièrement pures en anions nitrates. Plus précisément, la teneur en anions nitrates des dispersions, mesurée par la teneur en anions nitrates en poids des particules colloïdales est inférieure à 80ppm. Les dispersions de l'invention sont pures aussi en ce qui concerne leur teneur en ions chlorures.

La quantité d'élément M est généralement d'au plus 50%, cette quantité étant exprimé par le rapport moles d'élément M/somme des moles d'élément M et de cérium. L'élément M peut être présent sous différents états d'oxydation. L'invention s'applique bien entendu aux dispersions contenant plusieurs éléments M.

Les dispersions de l'invention peuvent présenter en outre un pH élevé, par exemple compris entre 5 et 8. Ces valeurs de pH, voisines de la neutralité, permettent des applications intéressantes des dispersions de l'invention.

Les particules colloïdales qui constituent les sols de l'invention sont fines. Ainsi, elles peuvent présenter un diamètre moyen qui peut être compris notamment entre 2 et 6nm. Ce diamètre est déterminé par comptage photométrique à partir d'une analyse par METHR (Microscopie Electronique par Transmission à Haute Résolution).

Le procédé de préparation des dispersions de l'invention va maintenant être décrit.

Ce procédé comprend une première étape dans laquelle on fait réagir avec une base un mélange d'au moins un sel de cérium avec au moins un sel d'un élément M. On peut partir notamment d'un sel de cérium III ou d'un mélange comprenant un sel de cérium IV en plus du sel de cérium III.

Comme base, on peut utiliser notamment les produits du type hydroxyde. On peut citer les hydroxydes d'alcalins ou d'alcalino-terreux et l'ammoniaque. On peut aussi utiliser les amines secondaires, tertiaires ou quaternaires. Toutefois, les amines et l'ammoniaque peuvent être préférés dans la mesure où ils diminuent les risques de pollution par les cations alcalins ou alcalino terreux. On peut aussi mentionner l'urée.

Comme sels de cérium III, on peut utiliser plus particulièrement l'acétate, le chlorure ou le nitrate de cérium III ainsi que des mélanges de ces sels comme des mixtes acétate/chlorure. Pour le cérium IV, on peut utiliser le nitrate de cérium IV et pour les autres éléments les chlorures et les nitrates notamment. On peut utiliser des sels du même type pour le ou les autres éléments M.

Selon une caractéristique spécifique du procédé de l'invention, la réaction du sel de cérium avec la base se fait en présence d'un acide.

Comme acides susceptibles d'être utilisés, on peut mentionner les acides minéraux et plus particulièrement ceux correspondant aux sels de cérium , notamment de cérium III, utilisés dans la réaction. On peut ainsi citer notamment l'acide acétique, l'acide nitrique ou l'acide chlorhydrique.

Il faut noter que l'acide peut aussi être apporté par la solution d'un sel dans laquelle il est incorporé. Par exemple, on peut utiliser comme solution de départ une solution de chlorure de titane acide comme TiOCl₂, 2HCl.

La quantité d'acide présent ou mis en oeuvre lors de la réaction est telle que le rapport atomique H⁺/(Ce+M) soit supérieur à 0, 1, de préférence 0,25.

La réaction de la base avec les sels peut se faire en continu, on entend par là une addition simultanée des réactifs dans le milieu réactionnel.

Le pH du milieu réactionnel est habituellement compris entre 7,5 et 9,5. On peut travailler dans des conditions telles que le pH du milieu réactionnel soit maintenu constant pendant la réaction.

On obtient à l'issue de la réaction précitée un précipité. Ce précipité peut être séparé du milieu liquide par tout procédé connu par exemple par centrifugation. Le précipité ainsi obtenu peut ensuite être remis en suspension dans l'eau de manière à donner la dispersion de l'invention. La concentration en cérium dans la dispersion ainsi obtenue est généralement comprise entre 0,005M et 2M de préférence entre 0,05M et 0,25 M.

Avantageusement, le précipité issu de la réaction peut être lavé. Ce lavage peut se faire en remettant dans l'eau le précipité puis, après agitation, en séparant le solide du milieu liquide par centrifugation par exemple. Cette opération peut être répétée plusieurs fois si nécessaire.

Selon une variante de l'invention, la dispersion obtenue après remise en suspension dans l'eau peut être purifiée et/ou concentrée par ultrafiltration.

Le lavage et l'ultrafiltration peuvent se faire sous air ou dans une atmosphère d'air et d'azote ou encore sous azote. L'atmosphère sous laquelle se déroulent ces opérations joue un rôle dans la transformation du cérium III en cérium IV.

Après la remise en suspension dans l'eau et après l'éventuelle étape de lavage et, de préférence, avant l'étape de concentration si une concentration est mise en oeuvre, il peut être avantageux de réaliser une oxydation de la dispersion; on améliore ainsi la stabilité de la dispersion. Ce traitement oxydant peut se faire de deux manières par exemple.

Une première manière consiste à maintenir la dispersion sous agitation et sous air, et ceci pendant une durée qui peut varier de 3 à 20 heures par exemple. La seconde manière consiste à ajouter de l'eau oxygénée à la dispersion. La quantité d'eau oxygénée ajoutée est réglée de manière à obtenir dans la dispersion finale le rapport CeIII/Ce total donné plus haut. Cette oxydation avec addition d'eau oxygénée est réalisée de préférence après une agitation sous air de la dispersion pour une durée supérieure à 2 heures. La durée de l'addition d'eau oxygénée peut être comprise entre 30 mn et 6 heures.

Les dispersions de l'invention peuvent être utilisées dans de nombreuses applications. On peut citer la catalyse notamment pour post combustion automobile, dans ce cas les dispersion sont utilisées dans la préparation de catalyseur. Les dispersions peuvent aussi être employées pour la lubrification, dans les céramiques, la fabrication de composés luminophores, dans la cosmétique et dans ce cas elles peuvent rentrer dans la préparation de compositions cosmétiques notamment dans la préparation de crèmes anti-UV. Elles peuvent être utilisées sur un substrat en tant qu'agents d'anticorrosion.

Des exemples vont maintenant être donnés. Dans ces exemples, la conductivité est mesurée à l'aide d'un conductimètre METROHM 660 CONDUCTOMETER équipé d'une cellule de conductivité TACUSSEL XE100. Les teneurs en Ce III sont données comme indiqué précédemment (rapport atomique CeIII/Ce total).

### EXEMPLE 1

Cet exemple concerne une dispersion colloïdale aqueuse de particules nanométriques de cérium et de titane à pH voisin de la neutralité. On additionne sous agitation 562,8 g de Ce(CH₃COO)₃ à 49,3% en CeO₂ (soit 1,6 moles de Ce) et 125 g de TiOCl₂, 2HCl à 3,19 mole/Kg de densité 1,56 (soit 0,4 moles en TiO₂). On complète à 3000ml par de l'eau déminéralisée. Le rapport molaire H⁺/(Ce+Ti) est de 0,4.

La précipitation du solide est réalisée dans un montage en continu comprenant :
- un réacteur d'un litre équipé d'un agitateur à pales, réglé à 400 t/mn avec un pied de cuve de 0,5 l et d'une électrode de contrôle;
- deux flacons d'alimentation contenant d'une part, la solution de sels de cérium précédemment décrite et d'autre part, une solution d'ammoniaque 3 N.

Le débit de la solution d'acétate de cérium et de TiOCl2 est fixée à environ 600ml/h et le débit de la solution d'ammoniaque est de 340ml/h. Ainsi, 2880ml du mélange de sels de cérium et de titane et 1630ml d'ammoniaque 3 N ont été ajoutés en 288 mn.

Le pH du milieu réactionnel est de 8,5 pendant toute la durée de la réaction.

On mesure un rendement de précipitation en Ce+Ti de 47%.

On obtient un précipité que l'on sépare par centrifugation.

Par calcination à 1000°C, on mesure que le précipité contient 15% d'oxyde de cérium et de titane.

Le précipité est dispersée par addition d'eau déminéralisée pour obtenir une dispersion à 0,12 M en Ce+Ti. On met sous agitation 15 mn. On centrifuge de nouveau. Deux opérations successives sont ainsi réalisées. La teneur en cérium III de la dispersion est de 60%. La dispersion est ensuite mise sous agitation sous atmosphère d'air, une nuit. A l'issue de ce traitement, la teneur en cérium III de la dispersion est de 6,5%, la teneur en cérium total est de 17,2g/l.

100ml de la dispersion à 0,1M en Ce+Ti sont dilués à 300ml par de l'eau déminéralisée. Par ultrafiltration sur des membranes 3 KD, on concentre jusqu'à 100ml. Trois ultrafiltrations sont ainsi réalisées. A la dernière ultrafiltration, on concentre jusqu'à obtenir une dispersion concentrée de concentration 5,7% en oxyde de cérium et de titane. Le pH est de 5,4 et la conductivité de 1,4mS/cm. La concentration en nitrate de la dispersion colloidale est inférieure à 80 ppm. Par cryométrie MET, on observe des particules nanométriques de taille d'environ 3 à 4 nm.

La dispersion obtenue est stable au moins 6 mois.

### EXEMPLE 2

Cet exemple concerne une dispersion colloïdale aqueuse de particules nanométriques de cérium et de fer à pH neutre.

On additionne sous agitation dans un bécher 307g de solution de nitrate de cérium (III) à 3M/I en Ce³⁺, de densité 1,715 et à H⁺ = 0,01 ( soit 0,537 mole Ce³⁺), puis 194,5g de solution de Ce(NO₃)₄ à Ce⁴⁺= 1,325M/I, H⁺=0,7 N, de densité 1,44 ( soit 0,179 moles Ce ⁴⁺), puis 73,8 g de Fe(NO₃)₃, 9H₂O à 98% ( soit 0,18 mole), préalablement dissous dans un volume total de 358ml ( solution à pH 1), puis 32,2 g d'acide acétique concentré Prolabo (soit 0,54 mole de CH₃COOH). On complète à 2000ml par de l'eau déminéralisée. L'ensemble est mis sous agitation jusqu'à obtention d'une solution limpide à l'oeil. Le mélange obtenu présente alors une concentration d'environ 0,45 M en cérium et fer.

La précipitation du solide est réalisée dans le montage en continu décrit à l'exemple 1.

Ainsi, 2000ml de la solution de sel de cérium et de fer et 800ml d'ammoniaque 3 N ont été additionnés en 240 mn.

La dispersion colloïdale obtenue après redispersion sous air du précipité est lavée par ultrafiltration par de l'eau déminéralisée préalablement ajustée à pH 7,5 puis concentrée par ultrafiltration jusqu'à obtenir une dispersion concentrée de concentration 10,5% en oxyde de cérium et de fer.

On obtient ainsi une dispersion colloïdale stable au moins 6 mois vis à vis de la décantation.

### EXEMPLE 3

Cet exemple concerne une dispersion colloïdale aqueuse de particules nanométriques de cérium et de lanthane à pH voisin de la neutralité.

On additionne sous agitation dans un bécher 512 g d'acétate de cérium à 49,3% en CeO₂, 208cm³ d'acide acétique concentré et on complète à 3000ml par de l'eau déminéralisée.

A 2500ml de cette solution d'acétate de Ce(III) contenant 1,225mole de Ce, on ajoute 500cm³ d'une solution d'acétate de La(III) à 0,57M en La soit 0,285 mole de La. Le rapport molaire H⁺/(Ce+La) est de 2,0.

La précipitation du solide est réalisée dans le montage en continu décrit à l'exemple 1.

Ainsi, 2670ml de la solution d'acétate de cérium-lanthane et 1971ml d'ammoniaque 3 N ont été ajoutés en 267mn.

Le pH du milieu réactionnel est de 8,5 pendant toute la durée de la réaction.

On mesure un rendement de précipitation en Ce+La de 85 %.

On obtient un précipité que l'on sépare par centrifugation.

Par calcination à 1000°C, le précipité est évalué à 21% en oxyde de cérium et de lanthane.

Le précipité est dispersé par addition d'eau déminéralisée pour obtenir une dispersion à 0,15M en Ce+La. On met sous agitation 15 mn. On centrifuge de nouveau. Deux opérations successives sont ainsi réalisées. La teneur en cérium III de la dispersion est de 60%. La dispersion est ensuite mise sous agitation sous atmosphère d'air, une nuit. A l'issue de ce traitement, la teneur en cérium III de la dispersion est de 5%.

100ml de la dispersion à 0,15 M en Ce+La sont dilués à 300ml par de l'eau déminéralisée. Par ultrafiltration sur des membranes 3 KD, on concentre jusqu'à 100ml. Trois ultrafiltrations sont ainsi réalisées. A la dernière ultrafiltration, on concentre jusqu'à obtenir une dispersion concentrée de concentration 15,5% en oxyde de cérium et de lanthane. Le pH est de 5,5 et la conductivité de 0,24mS/cm. La concentration en ions nitrates de la dispersion colloidale est inférieure à 80 ppm. Par cryométrie MET, on observe des particules nanométriques de taille d'environ 3 à 4 nm.

On obtient ainsi une dispersion colloïdale stable au moins 6 mois vis à vis de la décantation.

### EXEMPLE 4

Cet exemple concerne une dispersion colloïdale aqueuse de particules nanométriques de cérium et d'aluminium à pH voisin de la neutralité.

On additionne sous agitation dans un bécher 585 g d'acétate de cérium à 49,3% en CeO₂ (1,67 moles de Ce), 101 g d'AlCl₃, 9H₂O (M_{w}= 241 g/ mole, 0,42 mole d'Al) et 103 g d'HCl 10 M, et on complète à 3000 ml par de l'eau déminéralisée. Le rapport molaire H⁺/(Ce+Al) est de 0,5

La précipitation du solide est réalisée dans le montage en continu décrit à l'exemple 1.

Ainsi, 2440 ml de cette solution d'acétate de cérium-aluminium et 1580 ml d'ammoniaque 3 N ont été ajoutés en 244 mn.

Le pH du milieu réactionnel est de 8,5 pendant toute la durée de la réaction.

On détermine un rendement de précipitation de 64%

On obtient un précipité que l'on sépare par centrifugation.

Par calcination à 1000°C, le précipité est évalué à 8,1% en oxyde de cérium et d'aluminium.

Le précipité est dispersé par addition d'eau déminéralisée pour obtenir une dispersion à 0,25 M en Ce+Al. On met sous agitation 15 mn. On centrifuge de nouveau. Deux opérations successives sont ainsi réalisées. La teneur en cérium III de la dispersion est de 60%. La dispersion est ensuite mise sous agitation sous atmosphère d'air, une nuit. A l'issue de ce traitement, la teneur en cérium III est de 31%.

100 ml de la dispersion à 0,25 M en Ce+Al sont dilués à 300 ml par de l'eau déminéralisée. Par ultrafiltration sur des membranes de 3 KD, on concentre jusqu'à 100 ml. Trois ultrafiltrations sont ainsi réalisées. A la dernière ultrafiltration, on concentre jusqu'à obtenir une dispersion concentrée de concentration 10,6% en oxyde. Le pH de la dispersion est de 6.

On obtient ainsi une dispersion colloïdale stable au moins 6 mois vis à vis de la décantation.

## Revendications

1. Dispersion colloïdale aqueuse d'un composé de cérium et d'au moins un autre élément M choisi parmi le titane, le vanadium, le chrome, le manganèse, le fer, le cobalt, le nickel, le cuivre, le zinc, l'aluminium, le gallium, le zirconium et le groupe des terres rares constitué par l'yttrium et les éléments de la classification périodique de numéro atomique compris inclusivement entre 57 et 71 à l'exception du cérium, le composé du cérium et de l'élément M étant un oxyde ou un oxyde hydraté, **caractérisée en ce qu'**elle présente une conductivité d'au plus 5mS/cm.

2. Dispersion selon la revendication 1, **caractérisée en ce qu'**elle présente une concentration d'au moins 50g/l, plus particulièrement d'au moins 80g/l, cette concentration étant exprimée en oxyde et prenant en compte la somme des oxydes de cérium et du ou des éléments M.

3. Dispersion selon la revendication 1 ou 2, **caractérisée en ce qu'**elle contient du cérium III.

4. Dispersion selon la revendication 3, **caractérisée en ce qu'**elle présente un taux de cérium III par rapport au cérium total d'au plus 50%, plus particulièrement d'au plus 35%.

5. Dispersion selon l'une des revendications précédentes, **caractérisée en ce qu'**elle présente une quantité d'élément M d'au plus 50%, exprimé par le rapport moles d'élément M/somme des moles d'élément M et de cérium.

6. Dispersion selon l'une des revendications précédentes, **caractérisée en ce que** les particules colloïdales présentent une teneur en nitrate inférieure à 80ppm.

7. Dispersion selon l'une des revendications précédentes, **caractérisée en ce qu'**elle présente un pH compris entre 5 et 8.

8. Dispersion selon l'une des revendications précédentes, **caractérisée en ce qu'**elle présente une conductivité d'au plus 2mS/cm et plus particulièrement d'au plus 1mS/cm.

9. Dispersion selon l'une des revendications 1 à 8, **caractérisée en ce qu'**elle présente une conductivité inférieure à 0,3mS/cm.

10. Procédé de préparation d'une dispersion colloïdale selon l'une des revendications précédentes, **caractérisé en ce qu'**on fait réagir avec une base un mélange d'au moins un sel de cérium avec au moins un sel d'un élément M précité et en présence d'un acide en quantité telle que le rapport atomique H⁺/(Ce+M) soit supérieur à 0,1, de préférence 0,25, le pH du milieu réactionnel étant compris entre 7,5 et 9,5; puis on lave et on redisperse dans l'eau le précipité issu de la réaction précédente.

11. Procédé selon la revendication 10, **caractérisé en ce qu'**après la redispersion dans l'eau du précipité, on purifie par ultrafiltration la dispersion obtenue.

12. Procédé selon la revendication 10 ou 11, **caractérisé en ce que** le sel de cérium est un sel de cérium III, notamment un acétate ou un chlorure.

13. Procédé selon l'une des revendications 10 à 12, **caractérisé en ce qu'**on effectue la réaction en continu.

14. Procédé selon l'une des revendications 10 à 13, **caractérisé en ce qu'**après la redispersion dans l'eau du précipité, on fait subir un traitement oxydant à la dispersion.

15. Procédé selon la revendication 14, **caractérisé en ce qu'**on effectue le traitement oxydant soit par mise sous agitation sous air de la dispersion, soit par addition d'eau oxygénée.

16. Utilisation d'une dispersion du type selon l'une des revendications 1 à 9 ou du type obtenu par le procédé selon l'une des revendications 10 à 15, sur un substrat comme agent anticorrosion, dans une composition cosmétique, en catalyse notamment pour post combustion automobile, en lubrification ou dans les céramiques.

## Claims

1. Aqueous colloidal dispersion of a compound of cerium and at least one other element M chosen from among titanium, vanadium, chromium, manganese, iron, cobalt, nickel, copper, zinc, aluminium, gallium, zirconium, and the group of rare earths constituted by yttrium and the elements of the periodic classification having an atomic number of between 57 and 71 inclusive, with the exception of cerium, the compound of cerium and the element M being an oxide or a hydrated oxide, **characterized in that** it displays a conductivity of at most 5mS/cm.

2. Dispersion according to claim 1, **characterized in that** it has a concentration of at least 50 g/l, more particularly of at least 80 g/l, that concentration being expressed as oxide and taking into account the sum of cerium oxides and of element(s) M.

3. Dispersion according to claim 1 or 2, **characterized in that** it contains cerium III.

4. Dispersion according to claim 3, **characterized in that** it has a level of cerium III relative to the total cerium of at most 50%, more particularly of at most 35%.

5. Dispersion according to one of the preceding claims, **characterized in that** it contains a quantity of element M of at most 50%, expressed by the ratio moles of element M/sum of the moles of element M and of cerium.

6. Dispersion according to one of the preceding claims, **characterized in that** the colloidal particles have a nitrate content of less than 80 ppm.

7. Dispersion according to one of the preceding claims, **characterized in that** it has a pH of between 5 and 8.

8. Dispersion according to one of the preceding claims, **characterised in that** it displays a conductivity of at most 2mS/cm, more particularly of at most 1mS/cm.

9. Dispersion according to one of claims 1 to 8, **characterised in that** it displays a conductivity of less than 0.3mS/cm.

10. Process for preparing a colloidal dispersion according to one of the preceding claims, **characterized in that** there is reacted with a base a mixture of at least one salt of cerium with at least one salt of an element M mentioned above and in the presence of an acid in a quantity such that the atomic ratio H⁺/(Ce+M) is greater than 0.1, preferably 0.25, the pH of the reaction medium being between 7.5 and 9.5; then the precipitate from the preceding reaction is washed and re-dispersed in water.

11. Process according to claim 10, **characterized in that**, after the redispersion in water of the precipitate, the dispersion obtained is purified by ultrafiltration.

12. Process according to claim 10 or 11, **characterized in that** the salt of cerium is a salt of cerium III, in particular an acetate or a chloride.

13. Process according to one of claims 10 to 12, **characterized in that** the reaction is effected continuously.

14. Process according to one of claims 10 to 13, **characterized in that**, after the re-dispersion in water of the precipitate, the dispersion is subjected to an oxidative treatment.

15. Process according to claim 14, **characterized in that** the oxidizing treatment is effected either by stirring under air of the dispersion, or by addition of oxygenated water.

16. Use of a dispersion of the type according to one of claims 1 to 9 or of the type obtained by the process according to one of claims 10 to 15, on a substrate as an anticorrosion agent, in a cosmetic composition, in catalysis in particular for automobile post-combustion, in lubrication or in ceramics.

## Patentansprüche

1. Wäßrige kolloidale Dispersion einer Verbindung von Cer und mindestens einem weiteren Element M, ausgewählt aus Titan, Vanadium, Chrom, Mangan, Eisen, Cobalt, Nickel, Kupfer, Zink, Aluminium, Gallium, Zirkonium und der Gruppe der Seltenen Erden, bestehend aus Yttrium und den Elementen im Periodensystem mit den Atomzahlen zwischen 57 und 71 jeweils einschließlich mit Ausnahme von Cer, wobei die Verbindung aus Cer und dem Element M ein Oxid oder ein Oxidhydrat ist, **dadurch gekennzeichnet, daß** die Dispersion eine Leitfähigkeit von höchstens 5 mS/cm aufweist.

2. Dispersion nach Anspruch 1, **dadurch gekennzeichnet, daß** die Dispersion eine Konzentration von mindestens 50 g/l, insbesondere mindestens 80 g/l, aufweist, wobei diese Konzentration als Oxid berechnet ist und die Summe der Oxide von Cer und des oder der Elemente M umfaßt.

3. Dispersion nach Anspruch 1 oder 2, **dadurch gekennzeichnet, daß** die Dispersion Cer III enthält.

4. Dispersion nach Anspruch 3, **dadurch gekennzeichnet, daß** die Dispersion einen Gehalt an Cer III, bezogen auf die Gesamtmenge an Cer, von höchstens 50 %, insbesondere von höchstens 35 %, aufweist.

5. Dispersion nach einem der vorangehenden Ansprüche, **dadurch gekennzeichnet, daß** die Dispersion eine Menge an Element M von höchstens 50 % aufweist, berechnet als das Verhältnis der Mol von Element M zur Summe der Mol von Element M und Cer.

6. Dispersion nach einem der vorangehenden Ansprüche, **dadurch gekennzeichnet, daß** die kolloidalen Teilchen einen Nitratgehalt von weniger als 80 ppm aufweisen.

7. Dispersion nach einem der vorangehenden Ansprüche, **dadurch gekennzeichnet, daß** die Dispersion einen pH-Wert zwischen 5 und 8 aufweist.

8. Dispersion nach einem der vorangehenden Ansprüche, **dadurch gekennzeichnet, daß** die Dispersion eine Leitfähigkeit von höchstens 2 mS/cm, insbesondere höchstens 1 mS/cm, aufweist.

9. Dispersion nach einem der Ansprüche 1 bis 8, **dadurch gekennzeichnet, daß** die Dispersion eine Leitfähigkeit von weniger als 0,3 mS/cm aufweist.

10. Verfahren zur Herstellung einer kolloidalen Dispersion nach einem der vorangehenden Ansprüche, **dadurch gekennzeichnet, daß** man eine Mischung mindestens eines Cersalzes und mindestens eines Salzes eines vorgenannten Elementes M mit einer Base in Gegenwart einer Säure in einer solchen Menge, daß das H⁺/(Ce + M)-Atomverhältnis größer als 0,1, vorzugsweise größer als 0,25, ist, reagieren läßt, wobei der pH-Wert des Reaktionsmilieus zwischen 7,5 und 9,5 liegt, und man dann den aus der vorangehenden Reaktion stammenden Niederschlag (Präzipitat) wäscht und in Wasser redispergiert.

11. Verfahren nach Anspruch 10, **dadurch gekennzeichnet, daß** man nach Redispergieren des Niederschlags in Wasser die erhaltene Dispersion mittels Ultrafiltration reinigt.

12. Verfahren nach Anspruch 10 oder 11, **dadurch gekennzeichnet, daß** das Cersalz ein Cer(III)-Salz, insbesondere ein Acetat oder ein Chlorid, ist.

13. Verfahren nach einem der Ansprüche 10 bis 12, **dadurch gekennzeichnet, daß** man die Reaktion kontinuierlich durchführt.

14. Verfahren nach einem der Ansprüche 10 bis 13, **dadurch gekennzeichnet, daß** man nach Redispergieren des Niederschlags in Wasser die Dispersion einer Oxidationsbehandlung unterzieht.

15. Verfahren nach Anspruch 14, **dadurch gekennzeichnet, daß** man die Oxidationsbehandlung unter Rühren der Dispersion unter Luft oder aber durch Zugabe von Wasserstoffperoxid durchführt.

16. Verwendung einer Dispersion gemäß den Ansprüchen 1 bis 9 oder erhältlich durch das Verfahren nach einem der Ansprüche 10 bis 15 auf einem Substrat bzw. Träger als Korrosionsschutzmittel, in einer kosmetischen Zusammensetzung, in der Katalyse insbesondere zur Nachverbrennung im Kraftfahrzeugbereich, im Bereich der Schmiermittel oder in Keramiken.
